# EUROPÄISCHE PATENTSCHRIFT

(11) **EP 4 709 314 B1**
(45) Veröffentlichungstag und Bekanntmachung des Hinweises auf die Patenterteilung: **22.07.2026**
(21) Anmeldenummer: 25746664.9
(22) Anmeldetag: 28.07.2025
(51) Int. Cl.: A61B 50/20, A61B 50/30, A61B 50/33, A61C 3/04

(54) **AUFNAHME-EXPANDERHÜLSE SOWIE WERKZEUGLAGERUNGSSET**
RECEIVING EXPANDER SLEEVE AND TOOL STORAGE SET
MANCHON D'EXPANSION RÉCEPTEUR ET ENSEMBLE DE STOCKAGE D'OUTIL

(30) Priorität: 30.07.2024 DE 102024121708
(43) Veröffentlichungstag der Anmeldung: 18.03.2026
(73) Patentinhaber: Aesculap AG, 78532 Tuttlingen (DE)
(72) Erfinder: KNITTEL, Timo, 78573 Wurmlingen (DE); KLEMM, Svenja, 78187 Geisingen (DE)
(74) Vertreter: Winter, Brandl - Partnerschaft mbB
(86) Internationale Anmeldenummer: PCT/EP2025/071695
(87) Internationale Veröffentlichungsnummer: WO 2026/027486

(56) Entgegenhaltungen:
- EP-B1- 3 164 095
- WO-A1-2022/189410
- US-A1- 2017 143 449
- US-B2- 8 661 614
- US-B2- 8 701 246

## Beschreibung

### Technisches Gebiet

Die vorliegende Offenbarung betrifft eine Aufnahme-Expanderhülse für ein oder eines Werkzeuglagerungssets zur steckgelagerten Aufnahme chirurgischer Schaft-Werkzeuge sowie ein Werkzeuglagerungsset mit der Aufnahme-Expanderhülse und ggf. einer Halterungsvorrichtung.

### Hintergrund der Offenbarung

In der modernen Chirurgie kommt eine Vielzahl von verschiedenen Instrumentenköpfen/Werkzeugen wie z.B. Bohr- oder Fräswerkzeuge (mit Werkzeugschaft) zum Einsatz, die jeweils auf/in ein einen Instrumentenantrieb/Motor aufweisendes Handstück abnehmbar auf-/eingesteckt werden können. Dies erlaubt dem Operateur, während des Betriebs des Handstücks den auf dem / in das Handstück auf-/eingesteckten Instrumentenschaft mit distalem Instrumentenkopf/Effektor je nach Anforderung der Anwendung zu wechseln. Um dem Operateur hierbei einen möglichst einfachen Zugriff auf diese Instrumentenköpfe (nachstehend nur noch als Werkzeuge bezeichnet) zu ermöglichen, werden die Werkzeuge gewöhnlich in einem chirurgischen Werkzeuglagerungsset geordnet aufbewahrt, wie dies beispielsweise auch aus dem Handwerk beispielsweise für Bohrersets bekannt ist. Hierbei werden, um Beschädigungen der Werkzeuge zu vermeiden und ein rasches Auffinden eines benötigten Werkzeugs zu gewährleisten, die Werkzeuge jedoch nicht lose aufbewahrt, sondern in Aufnahme-Expanderhülsen in fixierender/eingespannter Weise aufgenommen, die wiederum in einer Lochmatrix des Werkzeugsets (im einfachsten Fall eine Lochplatte) eingesteckt bzw. darin fixiert sind.

Auf diese Weise sind die einzelnen Werkzeuge coaxial/parallelbeabstandet aneinandergereiht.

### Stand der Technik

Herkömmliche Werkzeuglagerungssets dieser Art für die Lagerung/Aufbewahrung von chirurgischen Werkzeugen gehören zum allgemeinen Stand der Technik. Diese bekannten Werkzeuglagerungssets weisen im Wesentlichen eine erste Lochplatte (ein sogenanntes Hülsenblech) auf, das als eine üblicherweise metallische Platte mit einer Lochmatrix aus runden Löchern unterschiedlicher Größe ausgebildet ist. In die Löcher sind Aufnahme-Expanderhülsen für die Aufnahme von Schaft-Werkzeugen eingesteckt / einsteckbar bzw. darin fixiert. Die Aufnahme-Expanderhülsen weisen jeweils eine Einstecköffnung für ein bestimmtes chirurgisches Werkzeug auf. Chirurgische Werkzeuge haben einen distalen Werkzeugeingriffsabschnitt (Effektor) und einen proximalen Schaftabschnitt, wobei Aufnahme-Expanderhülsen mit verschiedenen Durchmessern der Werkzeugaufnahmeöffnungen die korrekte und sitzgenaue Aufnahme von Werkzeugen mit verschiedenen Schaftdurchmessern erlauben. Zusätzlich kann das Werkzeugset eine mit der ersten Lochplatte verbundene zweite Lochplatte (ein sogenanntes Bodenblech) aufweisen, das parallel der ersten Lochplatte beabstandet ist und teilweise eine Einstecktiefe der chirurgischen Werkzeuge mitbestimmt.

Neben der schonenden Lagerung chirurgischer Werkzeuge ist die gründliche Reinigung und Sterilisation chirurgischer Werkzeuge von größter Wichtigkeit. Herkömmliche Aufnahme-Expanderhülsen sind als eine massive, zylindrische Kunststoffhülse mit elastisch radial-expandierbarem Inlay ausgebildet, wodurch das darin eingesteckte Werkzeug zwar sicher gehalten, jedoch eine ausreichende Umströmung der darin aufbewahrten Werkzeuge mit Reinigungsfluiden (z.B.: Reinigungsflüssigkeiten oder -gasen) erschwert oder gar verhindert wird.

Zur Verbesserung der Umströmung der Werkzeuge ist beispielsweise aus der EP 3 164 095 B1 eine Aufnahme-Expanderhülse bekannt, die einen mit radialen Durchbrüchen versehenen zylindrischen Hohlkörper aus Kunststoff sowie innerhalb des Hohlkörpers eingesetzte, nach innen ragende filigrane Klemmarme vorzugsweise aus Kunststoff oder Metall aufweist. Dabei dient der vergleichsweise massive zylindrische Kunststoffhohlkörper zum Befestigen der Aufnahme-Expanderhülse an einer eine Lochplatte aufweisenden Haltungsvorrichtung, wohingegen die filigranen Klemmarme zum punktartigen Kontaktieren des in die Aufnahme-Expanderhülse eingesteckten Werkzeugs mit möglichst geringer Kontaktfläche dienen.

Aus der US 2017/0143449 A1 ist ebenfalls eine Aufnahme-Expanderhülse bekannt, mit einem zylindrischen äußeren Hohlkörper und einem inneren Werkzeug-Halteeinsatz. Der zylindrische äußere Hohlkörper weist an seiner einen Stirnseite einen äußeren, umlaufenden Kragen auf, der als Anschlagsflansch dient, um beim Einstecken der Aufnahme-Expanderhülse in die Lochplatte ein axiales Durchrutschen/ Durchstecken des äußeren Hohlkörpers zu vermeiden.

US 8661614 B2 offenbart eine Aufnahme-Expanderhülse die eine Basisstruktur und eine obere Struktur umfasst, die mit der Basisstruktur durch eine Mehrzahl von Seitenwänden verbunden ist, wobei innerhalb der Mehrzahl der Seitenwände eine Öffnung mit einer zentralen Achse ausgebildet ist.

Zwar zeichnen sich diese bekannten Aufnahme-Expanderhülsen durch eine hohe Stabilität aus, haben jedoch auch weiterhin den Nachteil, dass der Kunststoffhohlkörper zwar radiale Durchbrüche aufweist, um das Eindringen und Umspülen des Reinigungsfluids zu ermöglichen, es aber immer noch möglich ist, dass sich beispielsweise Gewebereste und dergleichen Verunreinigungen an der Aufnahme-Expanderhülse ansammeln. Ein weiterer entscheidender Nachteil an diesen bekannten Aufnahme-Expanderhülsen liegt zudem darin, dass die Kunststoffhohlkörper in der Herstellung, Montage und Bestückung teuer und aufwändig sind.

Um hohen Temperaturen bei der Sterilisation widerstehen zu können, müssen die Kunststoffteile aus speziellem temperaturresistentem Kunststoff gefertigt sein, was die Fertigungskosten erhöht. Um diese medizinische Anforderung erfüllen zu können, wird in der Regel der Werkstoff PEEK verwendet, der als solcher kostenintensiv und schwer zu verarbeiten ist sowie eine begrenzte Lebensdauer aufweist. Zudem ist es bei einem mehrteiligen Aufbau der Aufnahme-Expanderhülse, insbesondere mit dem Kunststoffhohlkörper und den darin eingelassenen oder eingesetzten Metallklemmarmen, erforderlich, die einzelnen Bestandteile in manueller Handarbeit zusammenzuführen. Dies ist nicht nur zeitaufwändig, sondern birgt auch das Risiko der Beschädigung der Aufnahme-Expanderhülsen oder der fehlerhaften Zusammenführung bei der Montage. Zudem bleiben Verschmutzungen an Kunststoff aufgrund dessen positiver Oberflächenladung oft länger haften als an anderen Werkstoffen wie beispielsweise Metall, was die Reinigung der Aufnahme-Expanderhülse zusätzlich erschwert. Auch bei der Trocknung des Werkzeugsets nach der Reinigung erweist sich der massive Aufbau herkömmlicher Werkzeugsets und deren Fertigungsmaterial Kunststoff als nachteilig.

Die in den Kunststoffhohlkörper eingesetzten Metallklemmarme der aus dem Stand der Technik bekannten Werkzeuglagerungssets und Aufnahme-Expanderhülsen lassen sich jedoch ohne die Kunststoffhohlkörper nicht oder nicht stabil an der Halterungsvorrichtung der bekannten Werkzeugsets befestigen.

Schließlich ist aus der WO 2022/189410 A1 ein Werkzeuglagerungsset bekannt mit einer Aufnahme-Expanderhülse, an der neben den metallischen Klemmarmen zum Haltern des Werkzeugs entweder an zusätzlichen (weiteren/separaten) metallischen Befestigungsarmen oder an jeweils einem Axialabschnitt der metallischen Klemmarme selbst Befestigungsabschnitte ausgebildet sind, um alleine daran die Aufnahme-Expanderhülse an der Halterungsvorrichtung/Lochplatte zu befestigen. Diese Befestigungsabschnitte zeichnen sich insbesondere in der Weise aus, dass sie elastisch verformbar (weil metallisch) sowie radial gekrümmt oder (radial nach außen) abgewinkelt ausgebildet sind, so dass sie (aufgrund ihrer Elastizität) radial klemmend (und krallend) in die Halterungsvorrichtung/Lochplatte eingesetzt werden können und in ihrem radial eingeklemmten Zustand durch ihre radiale Krümmung oder Abwinkelung die Halterungsvorrichtung / Lochplatte entgegen einer Einsteckrichtung des Werkzeugs hintergreifen können, so dass sie sowohl (radial) kraftschlüssig als auch (axial) formschlüssig an der Halterungsvorrichtung befestigbar sind.

Dies bedeutet, dass die aus dem vorstehend zuletzt genannten Stand der Technik bekannte Aufnahme-Expanderhülse auf einen zylindrischen äußeren Hohlkörper aus Kunststoff zur Befestigung der Aufnahme-Expanderhülse in der Lochplatte komplett verzichtet und stattdessen das bisherige Metall-Inlay zur klemmenden Halterung eines Werkzeugs selbst mit Befestigungsarmen oder Befestigungsabschnitten ausbildet. Diese Ausgestaltung erfordert aber nach wie vor eine metallische Ausführung der Aufnahme-Expanderhülse zur Erreichung ausreichender Stabilität.

### Kurzbeschreibung der Offenbarung

Es ist angesichts des vorstehenden Stands der Technik die Aufgabe der vorliegenden Offenbarung, eine Aufnahme-Expanderhülse für ein oder eines Werkzeuglagerungssets sowie ein Werkzeuglagerungsset bereitzustellen, welche einfach und kostengünstig herzustellen sind, eine effiziente Reinigung und/oder Sterilisation gewährleisten sowie eine einfach aufgebaute und gleichzeitig stabile Befestigung der Aufnahme-Expanderhülse an einer Halterungsvorrichtung des Werkzeuglagerungssets ermöglichen.

Die Aufgabe der vorliegenden Offenbarung wird durch eine Aufnahme-Expanderhülse für ein oder eines Werkzeuglagerungssets mit den Merkmalen des Patentanspruchs 1 sowie durch ein Werkzeuglagerungsset mit den Merkmalen des nebengeordneten Patentanspruchs gelöst. Vorteilhafte Weiterbildungen sind Gegenstand der Unteransprüche.

Der Kern der Offenbarung besteht demzufolge darin, dass für die Aufnahme-Expanderhülse nunmehr lediglich ein (äußerer) Hohlkörper aus Kunststoff, vorzugsweise ein Silikon-Kunststoff vorgesehen ist (und daher kein, aus dem Stand der Technik bekanntes metallisches Inlay eingesetzt ist), an dem sowohl zumindest ein Befestigungsabschnitt (eine Anzahl von Befestigungsabschnitten zum In-Eingriff-Kommen mit einer Halterungsvorrichtung / Lochplatte sowie eine Anzahl von Klemmbereichen, bzw. Klemmvorsprüngen oder Klemmarmen (stoffeinstückig) ausgebildet/ausgeformt sind, über die eine Klemmkraft auf ein eingestecktes Schaft-Werkzeug ausgeübt wird. Zur Erreichung der erforderlichen Stabilität der Aufnahme-Expanderhülse hat der Kunststoffhohlkörper (insbesondere Silikon-Hohlkörper, der nachfolgend auch als Silikon-Hülse bezeichnet wird) eine (zumindest äußere, vorzugsweise auch innere) Trichter-, insbesondere Kegelstumpf-Form mit einer großdurchmessrigen und einer kleindurchmessrigen Stirnseite/Stirnplatte, wobei die beiden (axial parallel-beabstandeten) Stirnseiten/Stirnplatten durch eine Mehrzahl von umfangsbeabstandeten Längs-/ Axialstäben (im Wesentlichen umfangsrandseitig) miteinander verbunden sind.

Die kleindurchmessrige Stirnseite/Stirnplatte stellt einen Werkzeugaufstandsboden dar und ist hierfür bevorzugt als geschlossene Platte ausgebildet. Vorzugsweise kann sich diese kleindurchmessrige Stirnplatte quasi zwangsläufig auch ausbilden, indem die Längs-/ Axialstäbe an ihren jeweiligen Axialenden (radial) zusammengeführt und miteinander verbunden (verschmolzen, vergossen, etc.) werden/sind. Ein von den Längs-/ Axialstäben definierter Innenumfang im Axialbereich der kleindurchmessrigen Stirnplatte (einschließlich unmittelbar oberhalb der kleindurchmessrigen Stirnplatte) kann ggf. ferner einen ersten, radial einwirkenden Klemmabschnitt für ein eingestecktes Schaft-Werkzeug bilden. Die großdurchmessrige Stirnplatte hat ein zentrales Durchgangs-/ Werkzeug-Einsteckloch mit einer Anzahl/Mehrzahl von umfangsbeabstandeten, radial nach innen vorragenden Klemmbereichen in Form von (Klemm-)Vorsprüngen als zweiter Klemmabschnitt und mit einer äußeren Umfangsnut (geschlossen oder als umfangsbeabstandete Nutabschnitte ausgebildet) als Befestigungsabschnitt(e) zur Befestigung der Aufnahme-Expanderhülse in der Haltevorrichtung / Lochplatte.

Es kann vorgesehen sein, dass die Aufnahme-Expanderhülse eine Anzahl von Erhebungen innerhalb der Befestigungsnut aufweist, die in vorzugsweise gleichmäßigem Umfangsabstand angeordnet sind und die dafür vorgesehen sind, jeweils einen Punktkontakt von der Nut zur Lochplatte zu erzeugen.

Der von den (Klemm-)Vorsprüngen definierte Innenradius ist dabei bevorzugt so bemessen, dass die (Klemm-)Vorsprünge bei Einstecken eines Schaft-Werkzeugs bestimmten Schaftdurchmessers radial deformiert werden, um so eine Klemmkraft auf das Schaft-Werkzeug auszuüben.

Vorzugsweise sind die radial nach innen vorragenden (Klemm-)Vorsprünge zur Oberseite der großdurchmessrigen Stirnplatte hin abgerundet, um ein Einstecken eines Schaft-Werkzeugs zu erleichtern und eine Beschädigung (Abreißen der Vorsprünge beim Einsteckvorgang) zu vermeiden.

Diese Ausgestaltung erbringt die folgenden Vorteile:
- Durch die Trichterform der Aufnahme-Expanderhülse lässt sich diese einfach ins Befestigungsloch der Haltevorrichtung / Lochplatte eindrücken bzw. von der gegenüberliegenden Seite der Lochplatte einziehen, bis diese in der/den Umfangsnut(en) als Befestigungsabschnitt(e) einrastet. Die Haltevorrichtung / Lochplatte stabilisiert somit umfangsseitig die großdurchmessrige Stirnplatte dergestalt, dass diese von einem eingesteckten Werkzeug über die Klemmvorsprünge nicht radial aufgeweitet werden kann. Dadurch ergibt sich die Klemmkraft im Wesentlich ausschließlich aus der elastischen Deformation der (Klemm-)Vorsprünge, wobei diese über die großdurchmessrige Stirnplatte bzw. die Nut(en) an den Lochrand der Lochplatte weitergegeben wird und somit eine stabile Befestigung der Aufnahme-Expanderhülse in der Lochplatte gewährleistet ist (Actio-Reactio-Prinzip).
- Die Nutenform des Befestigungsabschnitts hält die Aufnahme-Expanderhülse gleichsam in der Haltevorrichtung / Lochplatte sowohl bei Einstecken als auch Herausziehen eines Werkzeugs.
- Die in Axialrichtung ausgehend von der großdurchmessrigen Stirnplatte kegelförmig aufeinander zulaufenden Längs-/Axialstäbe bilden einen für eine Reinigungsflüssigkeit oder Dampf einfach zu durchdringenden Stabgitter-Käfig, sodass die Umströmung eines eingesteckten Werkzeugs mit Reinigungs-/Sterilisationsfluid gewährleistet ist.
- Die kleindurchmessrige Stirnplatte hält die Längs-/Axialstäbe unmittelbar oberhalb der kleindurchmessrigen Stirnplatte radial zusammen, sodass die Kegelstumpf-Form auch bei eingestecktem Schaft-Werkzeug beibehalten werden kann.
- Die Kleindurchmessrigkeit der den Hülsenboden ausbildenden Stirnplatte ist steif und stabil und daher besonders langlebig.
- Die Aufnahme-Expanderhülse kann vollständig aus Kunststoff, insbesondere Silikon-Kunststoff in einem Spritzgussverfahren hergestellt werden, was sehr preisgünstig ist.

Offenbarungsgemäß ist zudem ein medizinisches Werkzeuglagerungsset zur steckgelagerten Aufnahme medizinischer Schaft-Werkzeuge, mit der zuvor beschriebenen medizinischen Aufnahme-Expanderhülse und einer Halterungsvorrichtung vorgesehen, die als eine Lochplatte mit vorzugsweise runden Löchern zum Einstecken einer Anzahl von Aufnahme-Expanderhülsen ausgebildet ist, wobei die Lochplatte eine Plattenwanddicke hat, die der freien Spaltbreite der umlaufenden Nut der Aufnahme-Expanderhülse, insbesondere im Bereich der Erhebungen vorzugsweise mit einem bestimmten Übermaß in Nutenbreitenrichtung entspricht.

Die offenbarungsgemäße Aufnahme-Expanderhülse wird nachstehend anhand eines bevorzugten Ausführungsbeispiels unter Bezugnahme auf die begleitenden Figuren näher erläutert.

### Figurenbeschreibung

Fig. 1 zeigt drei unterschiedlich dimensionierte Aufnahme-Expanderhülsen gemäß der vorliegenden Offenbarung aus vier unterschiedlichen Richtungen,
Fig. 2 zeigt eine der drei Aufnahme-Expanderhülsen aus Fig. 1 in einem in einer Lochplatte eingebautem, sowie mit einem Werkzeug bestückten Zustand,
Fig. 3 zeigt die Aufnahme-Expanderhülse und Lochplatte aus Fig. 2 in einem Längsschnitt und

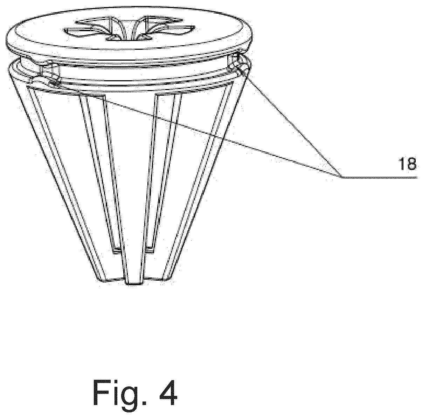

Fig. 4 zeigt eine Befestigungsnut zur Befestigung der Aufnahme-Expanderhülse aus Fig. 1 in einer Lochplatte in Vergrößerung.

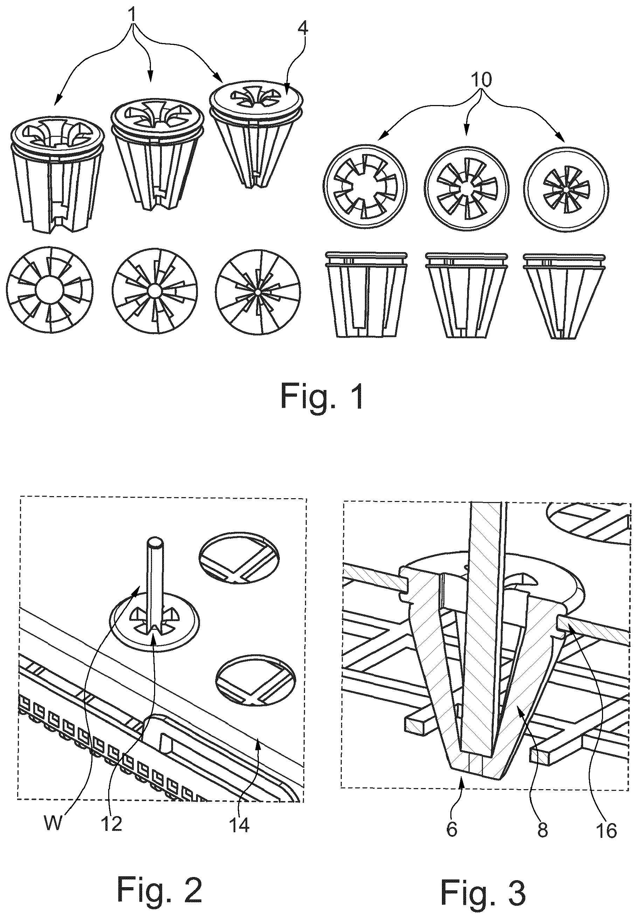

Gemäß der Fig. 1 hat eine Aufnahme-Expanderhülse 1 nach der vorliegenden Offenbarung eine obere, vorzugsweise kreisrunde (aber möglicherweise auch eckige) Stirnplatte (Ringscheibe) 4 zum Einstecken eines Schaft-Werkzeugs W, eine (vorzugsweise kreisrunde oder eckige) untere Stirnplatte 6 zum axialen Abstützen eines bereits eingesteckten Schaft-Werkzeugs W und einer Mehrzahl von Axial-/Längsstäben 8, die jeweils an den zueinander gerichteten Stirnplattenseiten (vorzugsweise im Bereich der jeweiligen Platten-Außenumfänge) der oberen und unteren Stirnplatten 4, 6 fixiert, angeformt oder ausgebildet sind, um diese miteinander zu verbinden. Die Axial-/ Längsstäbe 8 sind dabei in vorzugsweise gleichmäßigen Umfangsabständen voneinander beabstandet. Dadurch bilden sich schlitzartige Öffnungen oder Spalte zwischen den jeweils benachbarten Axial-/Längsstäben 8, sodass die Aufnahme-Expanderhülse 1 grundsätzlich eine siebkorbartige Gestalt erhält.

Die obere Stirnplatte 4 (nachfolgend auch als Einsteckplatte 4 bezeichnet) hat einen großen Durchmesser, wohingegen die untere Stirnplatte 6 (nachfolgend auch als Bodenplatte 6 bezeichnet) einen kleinen Durchmesser aufweist. Da die Axial-/Längsstäbe 8 jeweils im Umfangsrandbereich der beiden Platten 4, 6 an diesen (stoffeinstückig) fixiert sind, laufen die Axial-/Längsstäbe 8 ausgehend von der großdurchmessrigen Einsteckplatte 4 quasi zwangsläufig trichter-/kegelstumpfförmig in Richtung zur Bodenplatte 6 aufeinander zu.

Während die Bodenplatte 6 bevorzugt als geschlossene Platte ausgebildet ist, weist die Einsteckplatte 4 eine zentrale (Einsteck-)Öffnung 10 auf. Diese Einstecköffnung 10 ist in Form eines sogenannten Blumenausschnitts ausgebildet. In anderen Worten ausgedrückt hat die Einsteckplatte 4 insbesondere gemäß der Fig. 2 ein, vorzugsweise (gedachtes) kreisrundes, zentral angeordnetes Durchgangsloch, dessen Randbereich mit einer Mehrzahl von radial nach innen ragenden Vorsprüngen 12 (stoffeinstückig) bestückt ist, die gemeinsam einen Innendurchmesser definieren. Der Innendurchmesser ist geringfügig kleiner als der Schaftdurchmesser eines zu erwartenden medizinischen Schaft-Werkzeugs, wodurch ein eingestecktes medizinisches Schaft-Werkzeug von den Vorsprüngen 12 umfangsseitig eingeklemmt wird.

Die Vorsprünge 12 sind insbesondere gemäß der Fig. 1 bevorzugt an jenen Winkelpositionen zwischen den Axial-/Längsstäben 8 platziert (im Bereich der Spalte/Durchbrüche) und entsprechen daher anzahlmäßig vorzugsweise den Axial-/Längsstäben 8. Jeder der vorzugsweise steg-/leistenförmigen Vorsprünge 12 ist an seinem oberen, d.h. der Bodenplatte 6 abgewandten, sowie frei nach innen vorragenden Seite/Kante/Ecke in Längsrichtung der Aufnahme-Expanderhülse 1 abgerundet, um ein Einstecken eines Schaft-Werkzeugs in das Durchgangsloch 10 zu vereinfachen und ein Abreißen der Vorsprünge 12 bei Einsteckvorgang zu vermeiden.

Die Vorsprünge 12 sind bevorzugt keilförmig (Zuspitzung in Richtung Innenseite) gestaltet und können an ihren radial inneren freien Enden/Rändern/Kanten abgeflacht sein (zur Ausbildung eines flächigen Aufstands am eingesteckten Schaft-Werkzeug) oder spitz/scharfkantig ausgebildet sein (zur Ausbildung eines im Wesentlichen schneiden- oder punktartigen Aufstands am eingesteckten Schaft-Werkzeug). Im ersten Fall (flächiger Aufstand) kann eine vergleichsweise hohe Klemmkraft auf ein eingestecktes Schaft-Werkzeug aufgebracht werden, wohingegen im zweiten Fall (spitzer/scharfkantiger Aufstand) eine verbesserte Benetzung eines eingesteckten Schaft-Werkzeugs mit Reinigungs-/Desinfektions-/Sterilisationsfluids erreicht werden kann, da der von den Vorsprüngen 12 abgedeckte Werkzeugschaftbereich vergleichsweise klein ist. Grundsätzlich ist es aber auch möglich, noppenförmige Vorsprünge vergleichbar zu halbkugelförmigen oder kegelförmigen Vorsprüngen vorzusehen.

Zur Montage der Aufnahme-Expanderhülse 1 an/in einer Lochplatte 14 ist die Einsteckplatte 4 der Aufnahme-Expanderhülse 1 gemäß der Fig. 3 mit einer geschlossen oder (in Umfngsrichtung) unterbrochen randseitig umlaufenden (Einrast-)Nut 16 ausgebildet mit einer Nutenbreite, die der zu erwartenden Lochplattenstärke angepasst ist, derart, dass die Lochplatte 14 unter Klemmspannung in die Umlaufnut 16 einrasten kann, wie dies ebenfalls in der Fig. 3 dargestellt ist. Dadurch wirkt die Lochplatte 14 wie eine starre Einfassung (ausschließlich) um die obere Einsteckplatte 4 herum und verhindert so ein radiales Aufweiten der Einsteckplatte 4 insbesondere beim Einsteckvorgang eines Schaft-Werkzeugs W.

An dieser Stelle sei darauf hingewiesen, dass eine optimale Umströmung der Aufnahme-Expanderhülse 1 sowie des darin eingesteckten Schaft-Werkzeugs W mit Reinigungs-/Sterilisationsfluid besondere Bedeutung für das Reinigungs-/Sterilisationsergebnis hat. Es daher zu gewährleisten, dass der Gesamtkontaktbereich zwischen Schaft-Werkzeug und Aufnahme-Expanderhülse, aber auch zwischen Aufnahme-Expanderhülse und Lochplatte möglichst klein gehalten wird.

Aus diesem Grund sind bevorzugt Erhebungen 18 in der umlaufenden (Einrast-)Nut 16 vorgesehen, die in weiter vorzugsweise gleichmäßigem Umfangsabstand angeordnet sind und die dafür sorgen sollen, dass möglichst kein flächiger Kontakt von Nut 16 zu Lochplatte14 entsteht, sondern nur ein Punktueller, um so ein besseres Durchspülen des Nutenspalts zu ermöglichen. Diese Erhebungen 18 sichern/schaffen also einen Abstand/Spülspalt zwischen der Oberseite der Lochplatte 14 und der oberen Flanke der Einrast-/Befestigungsnut 16 und/oder der Unterseite der Lochplatte 14 und der unteren Flanke der Einrast-/Befestigungsnut 16 und/oder zwischen der Lochmantelfläche der Lochplatte 14 und der Bodenfläche der Einrast-/Befestigungsnut 16. Es ist bevorzugt eine Mehrzahl (mindestens 3) solcher Erhebungen 18 vorgesehen.

Die Funktionsweise der offenbarungsgemäßen Aufnahme-Expanderhülse 1 lässt sich wie folgt zusammenfassen:
Die Aufnahme-Expanderhülse 1 gemäß dem bevorzugten Ausführungsbeispiel der Offenbarung besteht in Gänze (ausschließlich) aus einem Kunststoffmaterial, vorzugsweise einem Silikon-Kunststoff, der eine bestimmte/gewisse Eigenelastizität aufweist, die es erlaubt, die Expanderhülse 1 und insbesondere die Einsteckplatte 4 manuell elastisch zu verformen.

Durch die Trichter-/Kegelstumpfform der Aufnahme-Expanderhülse 1 kann diese einfach in ein Aufnahmeloch der Lochplatte 14 eingesteckt/eingezogen werden, wobei die trichterförmig ausgerichteten Axial-/Längsstäbe 8 als Einsteckführung dienen. Aufgrund der Eigenelastizität der (gesamten) Aufnahme-Expanderhülse 1 ist es auch möglich, deren Einsteckplatte 4 im Bereich der umlaufenden Nut 16 in den Lochrand der Lochplatte 14 einzudrücken/einzuziehen und somit die Aufnahme-Expanderhülse 1 in der Lochplatte 14 gegen eine weitere Axialverschiebung zu verrasten/verspannen.

Begünstigt wird dies insbesondere dadurch, dass beispielsweise der oberhalb der Lochplatte zu liegen kommende Wulst (obere Nutenflanke) an der Einsteckplatte 4 einen höheren Deformations-Widerstand gegen ein Durchschieben bzw. Durchziehen desselben durch ein Loch der Lochplatte 14 hat als der unterhalb der Lochplatte zu liegen kommende Wulst (untere Nutenflanke). "Oberhalb/unterhalb liegend" bezieht sich vorliegend jeweils auf den eingebauten Zustand der Aufnahme-Expanderhülse 1.

Dies verhindert, dass die ganze Aufnahme-Expanderhülse 1 unbeabsichtigt einmal komplett durch die Lochplatte 14 gezogen wird, wenn der Durchzugswiderstand des unterhalb liegenden Wulsts/Nutenflanke erst einmal überwunden wurde.

Um dies zu erreichen kann der Außendurchmesser des oberhalb liegenden Wulsts/Nutenflanke größer sein als der des unterhalb liegenden Wulsts/Nutenflanke (d.h. die obere Flanke der Befestigungsnut ist höher als die untere Flanke der Befestigungsnut) und/oder der obere Wulst ist in Axialrichtung gesehen dicker als der untere Wulst und/oder das Material im Bereich des oberen Wulsts hat einen höheren Elastizitätsmodul als das Material im Bereich des unteren Wulsts (dies kann durch unterschiedliche Materialien oder durch eine "steifere" Einlage z.B. in Form eines Kunststoff- oder Metallrings im oberen Wulst erreicht werden, evtl. ist dies auch durch eine Nachbearbeitung des oberen Wulsts realisierbar).

Wenn nunmehr ein medizinisches Schaft-Werkzeug W in die Aufnahme-Expanderhülse 1 eingesteckt werden soll, wird diese zunächst in das Einsteckloch 10 der Einsteckplatte 4 eingeführt, wobei die daran ausgebildeten Vorsprünge 12 elastisch deformiert werden und auf das Schaft-Werkzeug W als ein oberer Klemmabschnitt eine Klemm-/Haltekraft ausüben. Da die Vorsprünge 12 im Axial-Bereich der Einsteckplatte 4 platziert sind, welche wiederum an ihrem Umfang über die umlaufende Nut 16, vorzugsweise den darin angeordneten Erhebungen 18 radial an der Lochplatte 14 radial abgestützt ist, wird hierbei die Einsteckplatte 4 nicht (geringfügig) radial aufgeweitet, sodass trotz des Silikon-Kunststoffmaterials am oberen Klemmabschnitt eine ausreichende Klemmkraft auf das Schaft-Werkzeug W erzeugt werden kann. Gleichzeitig wird die Klemmkraft über die elastisch deformierbare Einsteckplatte 4 radial an den Lochrand der Lochplatte 14 weitergegeben (Actio-Reactio-Prinzip) und bewirkt dort ein stabiles Verspannen der Einsteckplatte 4 in der Lochplatte 14.

Mit dem weiteren Einführen des medizinischen Schaft-Werkzeugs W kann dieses in Abhängigkeit seines Schaftdurchmessers irgendwann an seiner unteren Stirnkante mit den Innenseiten der kegelförmig zusammenlaufenden Axial-/Längsstäbe 8 in (Linien-)Kontakt kommen, wodurch sich ein axial zum oberen Klemmabschnitt versetzter unterer Klemmabschnitt quasi eigenständig ausbilden kann. Dadurch kann in dieser Konstellation zunächst ein weiteres Einführen des medizinischen Schaft-Werkzeugs W in die Aufnahme-Expanderhülse 1 behindert und ferner das eingeführte Schaftende umfangsseitig eingeklemmt (und damit der Schaft stabilisiert) werden. Wird in diesem Zustand das Schaft-Werkzeug W jedoch noch weiter in die Aufnahme-Expanderhülse 1 eingedrückt, kommt irgendwann der Werkzeugschaft mit der Bodenplatte 6 in axialen Anschlag, wodurch der Einführvorgang endgültig beendet ist.

Soll nunmehr das eingesteckte medizinische Schaft-Werkzeug W wieder aus der Aufnahme-Expanderhülse 1 herausgezogen werden, so kann sich ggf. die Gesamtklemmkraft aufgrund der Trichterform (über den Ausziehweg hinweg) verringern, nämlich indem beispielsweise zuerst die Klemmkraft am unteren Klemmabschnitt (nahe der Bodenplatte 6) aufgehoben wird, wodurch sich der Herausziehvorgang insgesamt erleichtert. Dieser Effekt tritt natürlich immer nur dann ein, wenn der Schaftdurchmesser des eingesteckten Schaft-Werkzeugs W einen bestimmten Wert (in Abhängigkeit des Durchmessers der Bodenplatte) überschreitet. Sollte dort keine Klemmkraft erzeugt werden, etwa weil das eingesteckte Schaft-Werkzeug gegenüber der Bodenplatte zu kleindurchmessrig ist, fällt der Klemmeffekt im Bereich des unteren Klemmabschnitts natürlich weg.

D.h. die Haupt-Klemmkraft auf ein eingestecktes Schaft-Werkzeug W wird grundsätzlich im oberen Klemmabschnitt (an der Einsteckplatte 4) erzeugt, wohingegen eine Neben-Klemmkraft im unteren Klemmabschnitt (nahe der Bodenplatte 6) in Abhängigkeit der Durchmesser des Werkzeugschafts und der Bodenplatte nur ausnahmsweise eintritt und daher optional ist.

Da ferner die Einsteckplatte 4 von der umlaufenden Befestigungsnut umgeben ist, in welche die Lochplatte 14 radialstützend einrastet, bestimmt sich die auf das Schaft-Werkzeug W ausgeübte Klemmkraft im oberen Klemmabschnitt im Wesentlichen ausschließlich aus der Deformation der (Klemm-)Vorsprünge 12 und kann somit über deren Dimensionierung zuverlässig eingestellt werden.

Die vorliegende Offenbarung betrifft zusammengefasst eine Aufnahme-Expanderhülse 1 eines Werkzeuglagerungssets zur steckgelagerten Aufnahme medizinischer Werkzeuge W, mit zumindest einem Befestigungsabschnitt 16, der angepasst und vorgesehen ist, um unmittelbar mit einer Lochplatte 14 des Werkzeuglagerungssets zusammenzuwirken, wobei die Aufnahme-Expanderhülse 1 eine Trichter- oder Kegelstumpfform hat mit dem Befestigungsabschnitt 16 im großdurchmessrigen Bereich der Aufnahme-Expanderhülse 1, in welchem auch eine Werkzeug-Einstecköffnung (welche in die Aufnahme-Expanderhülse 1 hineinführt) angeordnet ist und wobei die gesamte Aufnahme-Expanderhülse 1 aus einem Kunststoff, vorzugsweise einem Silikon-Kunststoff gefertigt ist.

## Patentansprüche

1. Medizinische Aufnahme-Expanderhülse (1) für ein oder eines medizinischen Werkzeugsaufnahmesets zur steckgelagerten Aufnahme medizinischer Schaft-Werkzeuge (W), mit
• einer, ein Einsteckloch (10) aufweisenden Einsteckplatte (4) zum Einstecken eines medizinischen Schaft-Werkzeugs (W) in die Aufnahme-Expanderhülse (1),
• eine, axial von der Einsteckplatte (4) beabstandete Bodenplatte (6) zum axialen Abstützen eines bereits eingesteckten medizinischen Schaft-Werkzeugs (W),
• einer Mehrzahl von umfangsbeabstandeten Axial-/Längsstäben (8), die jeweils an den zueinander gerichteten Plattenseiten der Einsteck- und Bodenplatten (4, 6) fixiert, stoffeinstückig angeformt oder ausgebildet sind, derart, dass diese ausgehend von der Einsteckplatte (4) in Richtung zur Bodenplatte (6) trichter- oder kegelstumpfförmig aufeinander zulaufen,
• einer Mehrzahl von Klemm-Vorsprüngen (12), die am, von der Einsteckplatte (4) gebildeten Umfangsrand des Einstecklochs (10) angeordnet sind und radial nach innen vorragen und
• einer Befestigungsnut (16) zur Befestigung der Aufnahme-Expanderhülse (1) an/in einer Lochplatte (14), wobei die Befestigungsnut (16) am Umfangsrand der Einsteckplatte (4) ausgeformt ist und diese geschlossen oder in Abschnitten unterteilt umgibt.

2. Medizinische Aufnahme-Expanderhülse (1) nach Anspruch 1, **dadurch gekennzeichnet, dass** diese aus einem Kunststoffmaterial hergestellt ist.

3. Medizinische Aufnahme-Expanderhülse (1) nach einem der vorstehenden Ansprüche, **dadurch gekennzeichnet, dass** sich die Vorsprünge (12) radial nach innen keilförmig verjüngen und an ihren freien Enden eine Aufstandfläche, eine Aufstandskante oder einen Aufstandspunkt ausbilden.

4. Medizinische Aufnahme-Expanderhülse (1) nach Anspruch 3, **dadurch gekennzeichnet, dass** die Vorsprünge (12) in flächiger Verlängerung zur Außenseite der Einsteckplatte (4) ausgeformt sind und zur Aufstandsfläche, Aufstandsschneidkante oder Aufstandspunkt hin abgerundet sind.

5. Medizinische Aufnahme-Expanderhülse (1) nach einem der vorstehenden Ansprüche 1 bis 4, **dadurch gekennzeichnet, dass** die Vorsprünge (12) in Umfangsrichtung gesehen zwischen den Axial-/Längsstäben (8) platziert sind.

6. Medizinische Aufnahme-Expanderhülse (1) nach einem der vorstehenden Ansprüche 1 bis 5, **gekennzeichnet durch** eine Anzahl von Erhebungen (18) innerhalb der Befestigungsnut (16), die dafür vorgesehen sind, jeweils einen Punktkontakt von Nut (16) zu Lochplatte (14) zu erzeugen

7. Medizinische Aufnahme-Expanderhülse (1) nach Anspruch 6, **dadurch gekennzeichnet, dass** die Erhebungen (18) am Nutengrund und/oder an der in Einsteckrichtung gesehen, oberen Nutenflanke und/oder an der in Einsteckrichtung gesehen, unteren Nutenflanke angeordnet sind.

8. Medizinische Aufnahme-Expanderhülse (1) nach einem der vorstehenden Ansprüche 1 bis 7, **dadurch gekennzeichnet, dass** die in Einsteckrichtung gesehen, obere Nutenflanke einen höheren Deformationswiderstand besitzt als die der in Einsteckrichtung gesehen, untere Nutenflanke.

9. Medizinische Aufnahme-Expanderhülse (1) nach Anspruch 8, **dadurch gekennzeichnet, dass** die in Einsteckrichtung gesehen, obere Nutenflanke einen größeren Außensdurchmesser hat, und/oder dicker ist, und/oder aus einem steiferen Material besteht als die in Einsteckrichtung gesehen, untere Nutenflanke.

10. Medizinisches Werkzeuglagerungsset zur steckgelagerten Aufnahme medizinischer Schaft-Werkzeuge (W), mit einer medizinischen Aufnahme-Expanderhülse (1) nach einem der Ansprüche 1 bis 9, und einer Halterungsvorrichtung, die als eine Lochplatte (14) mit Löchern zum Einstecken einer Anzahl von Aufnahme-Expanderhülsen (1) aufweist, **dadurch gekennzeichnet, dass** die Lochplatte (14) eine Plattenwanddicke hat, die der freien Spaltbreite der umlaufenden Nut (16) der Aufnahme-Expanderhülse (1) entspricht.

## Claims

1. A medical receiving expander sleeve (1) for a or of a medical tool receiving set for receiving medical shaft tools (W) in a plug-mounted manner, with
• an insertion plate (4) having an insertion hole (10) for inserting a medical shaft tool (W) into the receiving expander sleeve (1),
• a base plate (6) spaced axially from the insertion plate (4) for axially supporting an already inserted medical shaft tool (W),
• a plurality of circumferentially-spaced, axial/longitudinal rods (8) which are each fixed, integrally formed or configured on the mutually facing plate sides of the insertion and base plates (4, 6) in such a manner that, starting from the insertion plate (4), they converge in a funnel-shaped or frustoconical manner in the direction of the base plate (6),
• a plurality of clamping projections (12) which are arranged on the circumferential edge of the insertion hole (10) formed by the insertion plate (4) and project radially inward, and
• a fastening groove (16) for fastening the receiving expander sleeve (1) on/in a perforated plate (14), wherein the fastening groove (16) is formed on the circumferential edge of the insertion plate (4) and surrounds the latter in a closed manner or divided into sections.

2. The medical receiving expander sleeve (1) according to claim 1, **characterized in that** it is produced from a plastic material.

3. The medical receiving expander sleeve (1) according to one of the preceding claims, **characterized in that** the projections (12) taper radially inward in a wedge-shaped manner and form a contact surface, a contact edge or a contact point at their free ends.

4. The medical receiving expander sleeve (1) according to claim 3, **characterized in that** the projections (12) are formed in a planar extension toward the outside of the insertion plate (4) and are rounded toward the contact surface, contact cutting edge or contact point.

5. The medical receiving expander sleeve (1) according to one of the preceding claims 1 to 4, **characterized in that** the projections (12) are placed between the axial/longitudinal rods (8) as viewed in the circumferential direction.

6. The medical receiving expander sleeve (1) according to one of the preceding claims 1 to 5, **characterized by** a number of elevations (18) within the fastening groove (16), wherein the elevations are provided in each case to produce a point contact from the groove (16) to the perforated plate (14).

7. The medical receiving expander sleeve (1) according to claim 6, **characterized in that** the elevations (18) are arranged on the groove base and/or on the upper groove flank as viewed in the insertion direction and/or on the lower groove flank as viewed in the insertion direction.

8. The medical receiving expander sleeve (1) according to one of the preceding claims 1 to 7, **characterized in that** the upper groove flank as viewed in the insertion direction has a higher deformation resistance than that of the lower groove flank as viewed in the insertion direction.

9. The medical receiving expander sleeve (1) according to claim 8, **characterized in that** the upper groove flank as viewed in the insertion direction has a larger outside diameter and/or is thicker and/or consists of a stiffer material than the lower groove flank as viewed in the insertion direction.

10. A medical tool mounting set for receiving medical shaft tools (W) in a plug-mounted manner, with a medical receiving expander sleeve (1) according to one of the claims 1 to 9, and a holding device which has a perforated plate (14) with holes for inserting a number of receiving expander sleeves (1), **characterized in that** the perforated plate (14) has a plate wall thickness which corresponds to the free gap width of the circumferential groove (16) of the receiving expander sleeve (1).

## Revendications

1. Manchon d'expansion récepteur (1) médical pour un ou d'un ensemble de logement d'outil médical pour le logement par enfichage d'outils à tige (W) médicaux avec
- une plaque d'enfichage (4) présentant un trou d'enfichage (10) pour l'enfichage d'un outil à tige (W) médical dans le manchon d'expansion récepteur (1),
- une plaque de fond (6) espacée axialement de la plaque d'enfichage (4) pour l'appui axial d'un outil à tige (W) médical déjà enfiché,
- une pluralité de barres axiales/longitudinales (8) espacées sur la circonférence qui sont fixées respectivement au niveau des côtés de plaque dirigés l'un vers l'autre des plaques d'enfichage et de fond (4, 6), formées ou configurées en un seul matériau de telle manière que celles-ci convergent l'une vers l'autre à partir de la plaque d'enfichage (4) en direction de la plaque de fond (6) en forme d'entonnoir ou de cône tronqué,
- une pluralité de saillies de serrage (12) qui sont agencées au niveau du bord circonférentiel du trou d'enfichage (10) formé par la plaque d'enfichage (4) et dépassent radialement vers l'intérieur et
- une rainure de fixation (16) pour la fixation du manchon d'expansion récepteur (1) au niveau/dans une plaque perforée (14), dans lequel la rainure de fixation (16) est formée au niveau du bord circonférentiel de la plaque d'enfichage (4) et entoure celle-ci de manière fermée ou divisée en sections.

2. Manchon d'expansion récepteur (1) médical selon la revendication 1, **caractérisé en ce que** celui-ci est fabriqué en un matériau plastique.

3. Manchon d'expansion récepteur (1) médical selon l'une quelconque des revendications précédentes, **caractérisé en ce que** les saillies (12) se rétrécissent radialement vers l'intérieur en forme de coin et forment une surface d'appui, une arête d'appui ou un point d'appui au niveau de leurs extrémités libres.

4. Manchon d'expansion récepteur (1) médical selon la revendication 3, **caractérisé en ce que** les saillies (12) sont formées en prolongement plat vers le côté extérieur de la plaque d'enfichage (4) et sont arrondies vers la surface d'appui, l'arête de coupe d'appui ou le point d'appui.

5. Manchon d'expansion récepteur (1) médical selon l'une quelconque des revendications précédentes 1 à 4, **caractérisé en ce que** les saillies (12) sont placées entre les barres axiales/longitudinales (8) vu dans le sens périphérique.

6. Manchon d'expansion récepteur (1) médical selon l'une quelconque des revendications précédentes 1 à 5, **caractérisé par** un nombre d'élévations (18) dans la rainure de fixation (16) qui sont prévues afin de générer respectivement un point de contact de la rainure (16) à la plaque perforée (14).

7. Manchon d'expansion récepteur (1) médical selon la revendication 6, **caractérisé en ce que** les élévations (18) sont agencées au niveau du fond de rainure et/ou au niveau du flanc de rainure supérieur vu dans le sens d'enfichage et/ou au niveau du flanc de rainure inférieur vu dans le sens d'enfichage.

8. Manchon d'expansion récepteur (1) médical selon l'une quelconque des revendications précédentes 1 à 7, **caractérisé en ce que** le flanc de rainure supérieur vu dans le sens d'enfichage possède une résistance à la déformation supérieure à celle du flanc de rainure inférieur vu dans le sens d'enfichage.

9. Manchon d'expansion récepteur (1) médical selon la revendication 8, **caractérisé en ce que** le flanc de rainure supérieur vu dans le sens d'enfichage présente un diamètre extérieur supérieur et/ou est plus épais et/ou se compose d'un matériau plus rigide que celui du flanc de rainure inférieur vu dans le sens d'enfichage.

10. Ensemble de logement d'outil médical pour le logement par enfichage d'outils à tige (W) médicaux, avec un manchon d'expansion récepteur (1) médical selon l'une quelconque des revendications 1 à 9 et un dispositif de maintien qui présente une plaque perforée (14) avec des trous pour l'enfichage d'un nombre de manchons d'expansion récepteurs (1), **caractérisé en ce que** la plaque perforée (14) présente une épaisseur de paroi de plaque qui correspond à la largeur de fente libre de la rainure circonférentielle (16) du manchon d'expansion récepteur (1).
